# FASCICULE DE BREVET EUROPEEN

(11) **EP 0 549 480 B1**
(45) Date de publication et mention de la délivrance du brevet: **27.08.1997**
(21) Numéro de dépôt: 92420395.3
(22) Date de dépôt: 03.11.1992
(51) Int. Cl.: A61F 2/40

(54) **Prothèse huméralemodulaire**
Modulare Humerusprothese
Modular humeral prosthesis

(30) Priorité: 27.12.1991 FR 9116445
(43) Date de publication de la demande: 30.06.1993
(73) Titulaire: TORNIER SA, F-38330 Saint Ismier (FR)
(72) Inventeur: Tornier, Alain, F-38330 Saint-Ismier (FR); Walch, Gilles, 69003 LYON (FR); Boileau, Pascal, 06300 NICE (FR)
(74) Mandataire: Monnier, Guy

(56) Documents cités:
- CH-A- 507 704
- DE-C- 3 600 804
- US-A- 4 919 670
- US-A- 5 002 581

## Description

La présente invention est relative à des perfectionnements apportés aux prothèses de l'extrémité supérieure de l'humérus, comprenant une queue qui s'ancre dans le canal huméral et une calotte sphérique propre à coopérer avec la glène de l'épaule.

La prothèse suivant l'invention associée à une glène prothétique permet le traitement chirurgical notamment des affections dégénératives de l'arthrose gléno-humérale, mais encore celui d'autres affections. De manière isolée, la prothèse humérale suivant l'invention est destinée à des indications larges telles que les fractures céphalo-tubérositaires non accessibles à une chirurgie conservatrice, mais aussi à tout syndrome douloureux de l'épaule lié à une destruction isolée de la tête humérale (ostéonécroses, etc...)

Bien entendu, on connaît déjà des prothèses destinées à de tels traitements telles que celles dites de NEER. Toutefois, une telle porthèse est monobloc, de telle sorte qu'il faut posséder en stock un grand nombre d'implants pour répondre aux exigences des divers patients. De plus, ces prothèses présentent une seule taille de tige et de calotte sphérique, cette dernière ne pouvant pas être déportée comme cela est nécessaire dans certains cas. De plus, cette calotte présente une inclinaison fixe et elle ne couvre pas de manière satisfaisante le plan de coupe de l'extrémité de l'humérus, de telle sorte qu'il faut adapter l'os à la prothèse avec toutes les conséquences anatomiques que cela entraîne.

On connaît aussi, d'après le document CH 507 704 une queue de prothèse de hanche qui comprend à l'une de ses extrémités une collerette pourvue d'une surface d'appui destinée à faire reposer ladite collerette sur le restant de la partie osseuse.

Un pivot est issu de la collerette en déterminant un angle d'inclinaison par rapport à la surface d'appui. Le pivot est de préférence conique et présente sur sa périphérie des moyens de retenue tels que des nervures, des dents, saillies ou analogues.

Sur cette queue de prothèse on peut venir fixer différentes rotules qui sont toutes solidaires d'un col prévu d'une inclinaison variable. La fixation est réalisée au moyen du pivot et des moyens de retenue. L'utilisation de plusieurs rotules permet de définir le plus favorablement l'angle d'inclinaison, l'angle de rétrotorsion et le déport latéral de la calotte sphérique.

Une telle prothèse comporte certains inconvénients en ce qui concerne la collerette qui est prévue d'une surface d'appui destinée à la faire reposer sur le restant de la partie osseuse empêchant à la prothèse un maintien optimum dans la métaphyse réduisant nettement le déplacement spatial de la calotte.

On connaît également d'après le brevet DE-C-3600804 une prothèse de hanche comportant une tige portée par un axe longitudinal qui est ancré dans le canal fémoral et dont l'extrémité supérieure est solidaire d'une collerette qui s'appuie sur la coupe osseuse. L'extrémité supérieure de la tige est percée d'un trou borgne à profil conique propre à recevoir le premier élément conique d'une entretoise. L'élément conique est porté par un axe incliné d'un angle par rapport à l'axe de la tige.

L'entretoise se substitue au col du fémur d'une prothèse de hanche classique qui est située à l'extérieur du fémur et au dessous de la collerette. L'entretoise comporte un second élément conique relié au premier par un coin dont l'inclinaison est variable.

L'élément conique est porté par un axe incliné d'un angle β par rapport à l'axe du premier élément conique. Le second élément conique est prévu pour recevoir une boule destinée à coopérer avec un cotyle prothétique.

Le coin présente des inclinaisons variables permettant de placer précisemment la boule dans l'espace. Le paramètre ainsi réglé est une rotation appelée antéversion sur le fémur qui est l'inclinaison entre l'axe de l'entretoise et l'axe de la tige.

L'axe du second élément conique de l'entretoise se confond avec celui de la boule. Ainsi, étant donné qu'il existe un seul et unique axe entre la boule et le second élément conique, il apparait difficile, voir même impossible, de pouvoir décaler angulairement la boule autour de son propre axe géométrique.

En outre on connaît d'après le brevet US 4919670 une prothèse d'épaule comportant une queue qui s'ancre dans le canal huméral et une calotte sphérique propre à coopérer avec la glène de l'épaule. Cette prothèse d'épaule ne comporte aucun moyen pour faire varier la position angulaire de la calotte autour de son axe géométrique excentré par rapport à son propre axe géométrique.

Les perfectionnements qui font l'objet de la présente invention visent à permettre la réalisation d'une prothèse humérale qui remédie aux inconvénients ci-dessus et qui permettent de plus d'obtenir :
a) un positionnement et un ancrage efficaces dans le canal huméral ;
b) la récupération de la hauteur totale de l'humérus reconstitué
c) un diamètre de la calotte humérale propre à assurer une couverture satisfaisante du plan de coupe de l'humérus;
d) un angle d'inclinaison satisfaisant de la calotte humérale ;
e) un angle de rétro-torsion de la calotte humérale convenable ;
f) un déport latéral de la calotte par rapport à l'axe huméral ;
g) un positionnement antéro-postérieur de la calotte par rapport à l'axe huméral ;
h) une hauteur appropriée de la calotte humérale.

Les paramètres e) et f) permettent une adéquation entre la prothèse et l'humérus quel que soit le côté, c'est-à-dire que la prothèse suivant l'invention peut être aussi bien appliquée à un bras gauche qu'à un bras droit.

A cet effet, la prothèse de l'extrémité supérieure de l'humérus suivant l'invention comprend :
- des moyens de faire varier la position angulaire de la calotte autour d'un axe géométrique excentré par rapport à son propre axe géométrique ;

Un avantage de la prothèse consiste en ce qu'elle comprend une face extrême d'une entretoise opposée à celle qui se trouve contre la face d'appui de la queue, constituant une collerette circulaire qui s'engage à jeu réduit dans un emboîtement excentré de la base de la calotte sphérique, tandis qu'un pion tronconique correspondant vient se coincer dans un alésage tronconique situé au centre de l'emboîtement de la calotte à la façon d'un cône Morse.

Un autre avantage consiste en ce que la calotte sphérique est pourvue d'une série de trous concentriques à l'emboîtement et dans l'un desquels s'engage un ergot porté par l'entretoise.

Le dessin annexé, donné à titre d'exemple, permettra de mieux comprendre l'invention, les caractéristiques qu'elle présente et les avantages qu'elle est susceptible de procurer :
Fig. 1 est une vue éclatée d'une prothèse établie conformément à l'invention ;
Fig. 2 est une vue de l'extrémité de la queue de la porthèse qui coopère avec l'entretoise ;
Fig. 3 est une vue de la face de l'entretoise qui coopère avec la queue de la prothèse suivant l'invention ;
Fig. 4 est une vue en plan de la face extrême de l'entretoise qui coopère avec la calotte sphérique ;
Fig. 5 est une vue par dessous de la calotte sphérique ;
Fig. 6 une coupe longitudinale d'une prothèse suivant l'invention à l'état monté et mise en place par rapport à l'extrémité supérieure de l'humérus.
Fig. 7 est une vue éclatée représentant une variante préférée de la prothèse suivant l'invention.

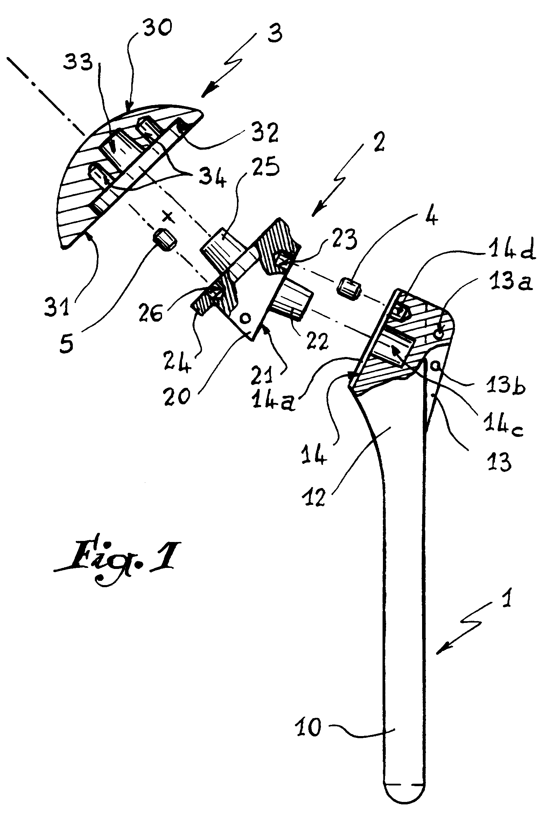

On a illustré en fig. 1 les trois éléments de la prothèse modulaire suivant l'invention soit une queue 1, une entretoise 2 et une calotte sphérique 3.

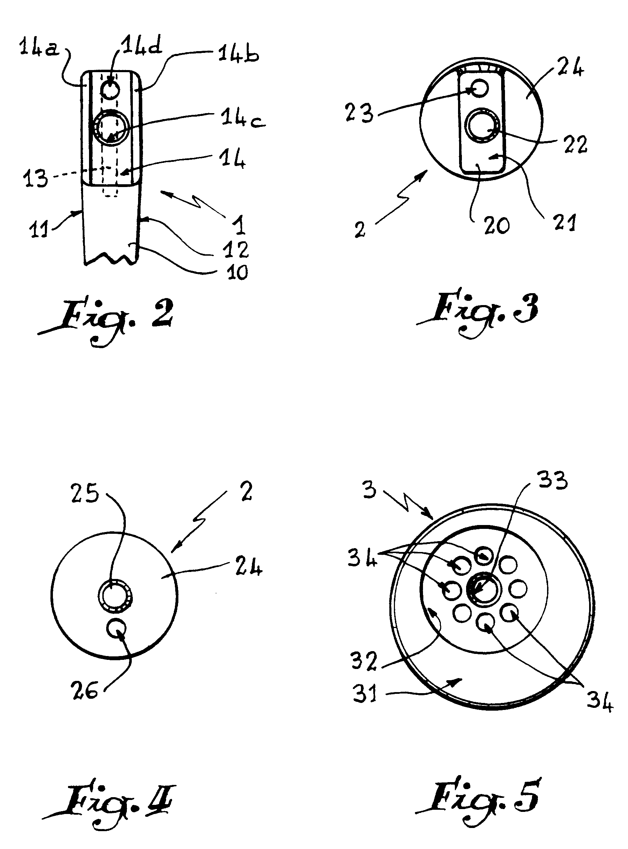

La queue 1 comprend une tige 10 de section cylindrique qui s'engage dans le canal huméral et dont la partie métaphysaire présente une partie externe rectiligne et une courbure interne. Dans le plan sagittal, cette partie métaphysaire présente une légère conicité latérale de ses flancs 11-12 (fig. 2). La partie métaphysaire comporte encore un aileron 13 disposé sur la face externe de la tige 10 et qui comprend deux perforations 13a-13b permettant, dans le cas de fracture, une reconstitution de l'extrémité supérieure de l'humérus autour de la prothèse.

La tige 10 se termine à l'opposé de l'aileron 13 par une face d'appui rectangulaire 14 inclinée par rapport à l'axe longitudinal de la tige 10 et dont les bords longitudinaux sont réalisés sous la forme de deux cordons rectilignes 14a, 14b. Au centre de la face d'appui 14 se trouve un alésage tronconique 14c au-dessus duquel est réalisé un trou borgne 14d de plus faible diamètre.

De par sa conception, la queue 1 s'adapte indifféremment du côté droit ou du côté gauche.

L'entretoise 2 est réalisée sous la forme d'un prisme 20 dont les deux faces extrêmes ne sont pas parallèles pour constituer une sorte de coin. La face extrême 21 de l'entretoise destinée à coopérer avec la face d'appui 14 de la queue est de largeur telle qu'elle vient s'insérer entre les deux cordons 14a, 14b de ladite face d'appui 14 avec un jeu réduit. Le centre de la face 21 est pourvu d'un pion tronconique 22 à côté duquel se trouve un trou borgne 23. Lors du montage, le pion 22 s'engage dans l'alésage 14c et comme la conicité de ces deux éléments est de l'ordre de 6°, cet assemblage constitue un cône de coincement dit cône Morse. Un téton 4 s'engage à ce moment dans les trous borgnes 14d et 23 pour parfaire l'assemblage.

La seconde face extrême de l'entretoise 2 est réalisée sous la forme d'une collerette circulaire 24 au centre de laquelle s'élève un pion tronconique 25 à côté duquel est ménagé un trou 26 destiné à recevoir un téton 5 semblable à celui 4 (fig. 4).

La calotte sphérique 3 comprend bien entendu une face extérieure 30 parfaitement polie limitée par une base 31. Dans cette dernière, on a ménagé de manière excentrée un emboîtement circulaire 32 dont le diamètre correspond au jeu près à celui de la collerette 24 de l'entretoise 2. Au centre de cet emboîtement est ménagé un alésage tronconique 33 de diamètre correspondant à celui du pion 25 de l'entretoise 2. Une série de huit trous 34 est réalisée dans le fond de l'emboîtement 32 de manière concentrique au centre de celui-ci. Le montage de la calotte sphérique 3 par rapport à l'entretoise 2 s'effectue par l'engagement du pion 25 dans l'alésage 33, la collerette 24 venant s'ajuster dans l'emboîtement 32, tandis que l'ergot 5 solidaire du trou 26 vient s'enfiler dans l'un des trous 34 à la position angulaire choisie de la calotte afin de fixer cette position. Bien entendu, par suite de la conicité d'environ 6° du pion 25 et de l'alésage 33, ces deux éléments s'assemblent par coincement.

Comme indiqué plus haut, on dispose d'une série d'entretoises de pentes différentes, de manière qu'on puisse régler l'inclinaison de la calotte sphérique humérale 3 suivant les cas cliniques. Bien entendu, toutes les entretoises présentent une face extrême 21 identique afin qu'elles puissent toutes s'adapter à la queue 1.

De même, on peut disposer d'un nombre conséquent de calottes sphériques de différents diamètres à chacun desquels correspond une hauteur donnée.

Grâce à la structure de la base de la calotte sphérique, on peut changer le positionnement antéro-postérieur de la calotte par rapport à l'axe huméral afin de s'adapter à toutes les positions nécessaires pour remplir la fonction normale de l'épaule traitée. De plus, grâce aux positions différentes de la calotte, on peut changer son positionnement anatomique de manière qu'elle puisse aussi bien être appliquée sur le côté gauche que sur te côté droit.

En bref, la structure de la calotte permet :
- le déport postérieur normal ;
- un positionnement respectant le point anatomique idéal ;
- la conservation de la rétro-torsion donnée par le positionnement de la tige humérale.

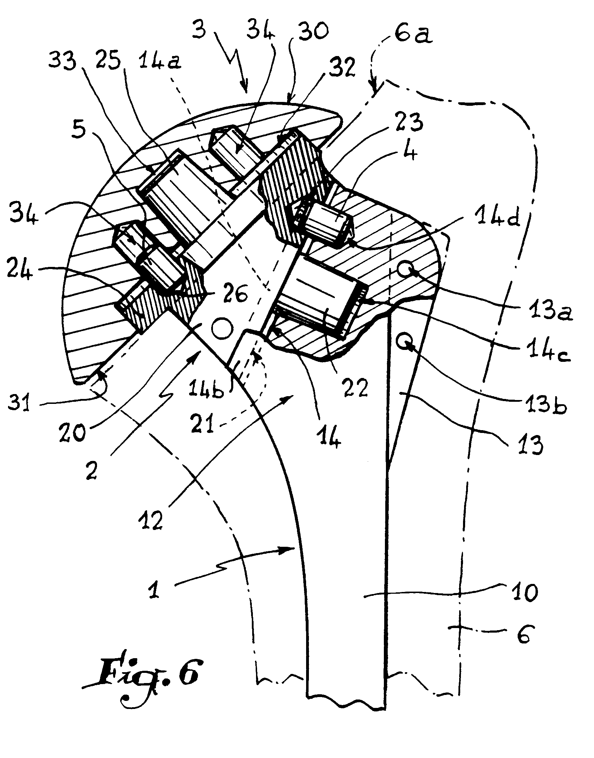

On observe en fig. 6 que la collerette 24 de l'entretoise vient reposer sur le plan 6a de coupe osseuse de l'humérus 6 qui a été silhouetté en traits discontinus.

On a ainsi réalisé une prothèse permettant de corriger tous les paramètres pour retrouver, en cas d'adaptation jugée incorrecte, un positionnement optimal de la calotte vis-à-vis de l'anatomie rencontrée.

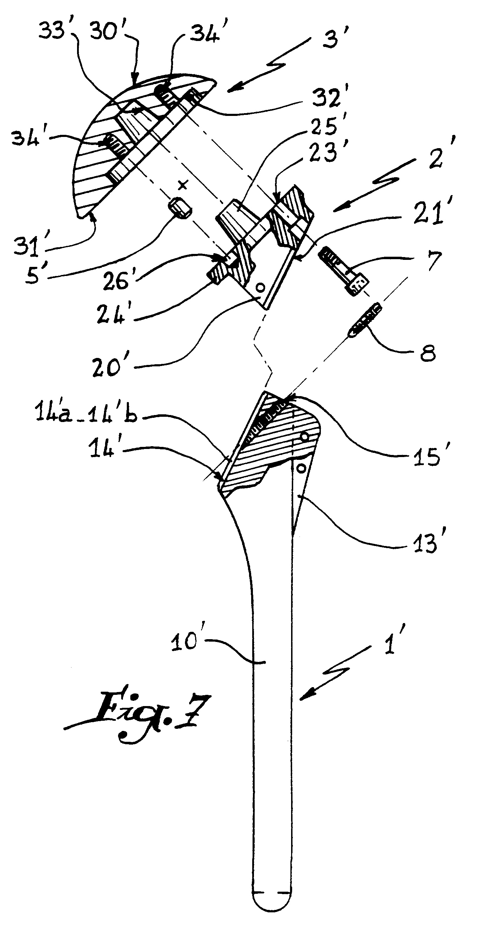

On a représenté en fig. 7 une variante préférée de la prothèse modulaire suivant l'invention soit une queue 1', une entretoise 2' et une calotte sphérique 3' .

La queue 1' comprend une tige 10' de section cylindrique qui s'engage dans le canal huméral et dont la partie métaphysaire présente une partie externe rectiligne et une courbure interne. La partie métaphysaire comporte un aileron 13' disposé sur la face externe de la tige 10' .

La tige 10' se termine à l'opposé de l'aileron 13' par une face d'appui rectangulaire 14' inclinée par rapport à l'axe longitudinal de la tige 10' et dont les bords longitudinaux sont réalisés sous la forme de deux cordons rectilignes 14'a, 14'b afin de constituer la mortaise d'une queue d'aronde. De plus un alésage 15' oblique et taraudé est ménagé de manière à venir déboucher d'une part au milieu de la face d'appui 14' et d'autre part dans la partie supérieure de la tige 10' à proximité de l'aileron 13' .

L'entretoise 2' est réalisée sous la forme d'un prisme 20' dont les deux faces extrêmes ne sont pas parallèles pour constituer une sorte de coin. La face extrême 21' de l'entretoise destinée à coopérer avec la face d'appui 14' de la queue présente la forme du tenon d'une queue d'aronde, ce qui permet de l'engager entre les deux cordons 14'a, 14'b de ladite face d'appui 14' avec j'eu réduit. L'entretoise 2' est percée d'un trou débouchant 23' étagé remplaçant le trou 22 de l'entretoise 2 et qui est prévu dans la partie la plus rétrécie de l'entretoise.

La seconde face extrême de l'entretoise 2' est réalisée, comme celle de la face correspondante de l'entretoise 2, sous la forme d'une collerette circulaire 24' au centre de laquelle s'élève un pion tronconique 25' à côté duquel est ménagé un trou 26' destiné à recevoir un téton 5' semblable à celui 5.

La calotte sphérique 3', en gros semblable à celle 3, comprend bien entendu une face extérieure 30' parfaitement polie limitée par une base 31' . Dans cette dernière, on a ménagé de manière excentrée un emboîtement circulaire 32' dont le diamètre correspond au jeu près à celui de la collerette 24' de l'entretoise 2' . Au centre de cet emboîtement est ménagé un alésage tronconique 33' de diamètre correspondant à celui du pion 25' de l'entretoise 2' . Une série de huit trous taraudés 34' est réalisée dans le fond de l'emboîtement 32' de manière concentrique au centre de celui-ci. Le montage de la calotte sphérique 3' par rapport à l'entretoise 2' s'effectue par l'engagement du pion 25' dans l'alésage 33', la collerette 24' venant s'ajuster, comme pour l'ensemble de fig. 1, dans l'emboîtement 32', tandis que l'ergot 5' solidaire du trou 26' vient s'enfiler dans l'un des trous 34' . L'immobilisation de la calotte 3' sur l'entretoise 2' est réalisée au moyen d'une vis 7 qui vient coopérer avec le trou taraudé 34', correspondant après avoir traversé le trou étagé 23' .

La calotte sphérique 3' étant montée sur l'entretoise 2', il suffit de fixer l'ensemble sur la queue 1' . Pour cela, la face extrême 21' de l'entretoise 2' est assemblée avec la face d'appui 14' de la tige 10' par l'intermédiaire du système à queue d'aronde. L'entretoise 2' est immobilisée sur la tige 10' au moyen d'une vis sans tête 8 qui vient appuyer contre la face extrême 21' de cette entretoise. Il va de soi que l'entretoise 2' peut avoir des positions variables par rapport à la face d'appui 14' par déplacement du tenon de la face 21', par rapport à la mortaise de la face 14' de la queue 1' .

Le verrouillage des deux assemblages s'effectue :
- soit totalement hors du champs opératoire, lors de l'assemblage des composants, pour certains cas d'indications tels ceux d'arthrose ,
- soit en deux temps pour les fractures, par exemple, à savoir après cimentage de la tige seule dans l'humérus :
   . assemblage et verrouillage de la calotte choisie sur l'entretoise déterminée, en dehors du champs opératoire, après les essais de sélection,
      puis
   . assemblage et verrouillage de cet attelage, sur la tige cimentée en place suivis par la reconstruction des fragments autour de la prothèse.

## Revendications

1. Prothèse de l'extrémité supérieure de l'humérus comprenant une queue (1,1') qui s'ancre dans le canal huméral et une calotte sphérique (3,3') unique propre à coopérer avec la glène de l'épaule et pourvue d'une entretoise (2,2') en forme de coin propre à faire varier la distance de la base de la calotte par rapport à la face d'appui de la queue, ladite entretoise permettant également de modifier l'orientation de la base de la calotte par rapport au plan contenant la face d'appui de la queue en changeant l'angle déterminé par ses faces extrêmes, caractérisée en ce qu'elle comprend des moyens (34,34',5) de faire varier la position angulaire de la calotte (3,3') autour d'un axe géométriquement excentré par rapport à son propre axe géométrique.

2. Prothèse suivant la revendication 1, caractérisée en ce que la face extrême (24) de l'entretoise (2) opposée à celle (21) qui se trouve contre la face d'appui (14) de la queue (1), constitue une collerette circulaire qui s'engage à jeu réduit dans un emboîtement excentré (32) de la base (31) de la calotte sphérique (3), tandis qu'un pion tronconique correspondant (25) vient se coincer dans un alésage tronconique (33) situé au centre de l'emboîtement (32) de la calotte (3) à la façon d'un cône Morse.

3. Prothèse suivant la revendication 2, caractérisée en ce que la calotte sphérique (3) est pourvue d'une série de trous (34) concentriques à l'emboîtement et dans l'un desquels s'engage un ergot (5) porté par l'entretoise (2).

4. Prothèse suivant la revendication 1, caractérisée en ce que les bords longitudinaux de la face d'appui (14) de la queue (1) sont réalisés sous la forme de deux cordons rectilignes (14a, 14b) entre lesquels vient s'insérer la face extrême correspondante (21) de l'entretoise (2).

5. Prothèse suivant la revendication 1, caractérisée en ce que les assemblages de l'entretoise (2) avec la calotte (3) et avec la queue (1) sont munis d'un système de verrouillage en position choisie.

6. Prothèse suivant la revendication 1, caractérisée en ce que les trous concentriques à l'emboîtement sont taraudés (34'), une vis d'immobilisation (7) s'engageant dans l'un des trous taraudés (34') après avoir traversé librement un trou (23') de l'entretoise (2').

7. Prothèse suivant la revendication 1, caractérisée en ce que la face extrême (21') de l'entretoise (2') est pourvue d'un tenon en queue d'aronde qui s'assemble avec une mortaise (14'a, 14'b) dans laquelle débouche un trou taraudé (15') dans lequel est vissé une vis d'immobilisation (8).

## Claims

1. Prosthesis for the upper end of the humerus, comprising a stem (1, 1') which anchors in the humeral canal, and a single spherical cap (3, 3') which is able to cooperate with the glenoid cavity of the shoulder and provided with a wedge-shaped spacing member (2, 2') which is able to vary the distance between the base of the cap and the bearing face of the stem, the said spacing member also making it possible to modify the orientation of the base of the cap in relation to the plane containing the bearing face of the stem by changing the angle defined by its end faces, characterized in that it comprises means (34, 34', 5) for varying the angular position of the cap (3, 3') about an axis which is geometrically off-centre in relation to its own geometric axis.

2. Prosthesis according to Claim 1, characterized in that the end face (24) of the spacing member (2), opposite that face (21) located against the bearing face (14) of the stem (1), constitutes a circular flange which engages, with reduced play, in an eccentric recess (32) in the base (31) of the spherical cap (3), while a corresponding frustoconical plug (25) wedges itself in a frustoconical bore (33) situated at the centre of the recess (32) of the cap (3) in the manner of a Morse cone.

3. Prosthesis according to Claim 2, characterized in that the spherical cap (3) is provided with a series of holes (34) concentric with the recess, and in one of which holes there engages a stud (5) borne by the spacing member (2).

4. Prosthesis according to Claim 1, characterized in that the longitudinal edges of the bearing face (14) of the stem (1) are made in the form of two rectilinear cords (14a, 14b) between which the corresponding end face (21) of the spacing member (2) is inserted.

5. Prosthesis according to Claim 1, characterized in that the assemblies of the spacing member (2) with the cap (3) and with the stem (1) are equipped with a system for locking in the chosen position.

6. Prosthesis according to Claim 1, characterized in that the holes concentric with the recess are tapped (34'), a fixing screw (7) engaging in one of the tapped holes (34') after it has passed freely through a hole (23') in the spacing member (2').

7. Prosthesis according to Claim 1, characterized in that the end face (21') of the spacing member (2') is provided with a dovetailed tenon which connects with a mortice (14'a, 14'b) into which there opens a tapped hole (15'), in which a fixing screw (8) is screwed.

## Patentansprüche

1. Prothese für das obere Ende des Humerus mit einem sich im Humeruskanal verankernden Stiel (1, 1') und mit einer einstückigen kalottenförmigen Kappe (3, 3'), die mit der Gelenkpfanne der Schulter zusammenwirkt, und mit einem winkelförmigen Distanzstück (2, 2'), das einen ungleichen Abstand der Grundfläche der Kappe zur Auflagefläche des Stiels ausbildet, wobei dieses Distanzstück auch ein Anpassen der Ausrichtung der Grundfläche der Kappe zu der durch die Auflagefläche des Stiels bestimmten Ebene über ein Ändern des durch dessen Außenflächen bestimmten Winkels erlaubt,
**gekennzeichnet durch**
Mittel (34, 34', 5), die die Winkelstellung der Kappe (3, 3') um eine zu deren eigentlichen Zentralachse geometrisch exzentrisch versetzten Achse variierbar halten.

2. Prothese nach Anspruch 1
**gekennzeichnet durch**
die Außenfläche (24) des Distanzstücks (2), die der der Auflagefläche (14) des Stiels (1) entgegengesetzt angeordneten Fläche (21) gegenüberliegt und die einen kreisförmigen Kragen ausbildet, der mit eingeschränkter Spieltoleranz in einen exzentrisch in der Grundfläche (31) der kalottenförmigen Kappe (3) angeordneten Rücksprung (32) einfaßt, wobei ein entsprechender kegelstumpfförmiger Zapfen (25) in eine in der Mitte des Rücksprungs (32) der Kappe (3) befindliche kegelstumpfförmige Bohrung (33) nach der Art eines Morsekegels zum Eingreifen kommt.

3. Prothese nach Anspruch 2
**gekennzeichnet durch**
die kalottenförmige Kappe (3), deren Rücksprung (32) eine Reihe von konzentrischen Löchern (34) aufweist, wobei in eines dieser Löcher ein von dem Distanzstück (2) getragener Stift (5) einfaßt.

4. Prothese nach Anspruch 1
**dadurch gekennzeichnet**,
daß die Längskanten der Auflagefläche (14) des Stiels (1) nach Art zweier geradliniger Nutränder (14a, 14b) ausgebildet sind, zwischen denen die entsprechende Außenfläche (21) des Distanzstücks (2) eingefügt wird.

5. Prothese nach Anspruch 1
**dadurch gekennzeichnet**,
daß die Verbindung des Distanzstücks (2) mit der Kappe (3) und mit dem Stiel (1) ein System zum Verriegeln in einer ausgewählten Position aufweist.

6. Prothese nach Anspruch 1
**dadurch gekennzeichnet**,
daß die konzentrischen Löcher im Rücksprung (32') mit Innengewinden (34') versehen sind, wobei in eines der Gewindelöcher (34') eine eine Bohrung (23') des Distanzstücks (2') frei durchquerende Sicherungsschraube (7) eingreift.

7. Prothese nach Anspruch 1
**dadurch gekennzeichnet**,
daß die Außenfläche (21') des Distanzstücks (2') eine Schwalbenschwanzzinke aufweist, die sich mit einer Führungsnut (14'a, 14'b) verbindet, in die eine Gewindebohrung (15') mündet, in der eine Sicherungsschraube (8) geschraubt ist.
